(19) Europäisches Patentamt
European Patent Office
Office européen des brevets

(11) Veröffentlichungsnummer: **0 392 037 A1**

(12) **EUROPÄISCHE PATENTANMELDUNG**

(21) Anmeldenummer: **89106362.0**

(22) Anmeldetag: **11.04.89**

(51) Int. Cl.[5]: **C07D 277/593, C07D 263/48, C07D 251/34, C07F 7/08**

(43) Veröffentlichungstag der Anmeldung:
**17.10.90 Patentblatt 90/42**

(84) Benannte Vertragsstaaten:
**AT CH DE FR IT LI NL**

(71) Anmelder: **TECHNOLOGITCHEN KOMBINAT ZA PROMISHLENA MIKROBIOLOGIA**

**Razgrad(BG)**

(72) Erfinder: **Atanassova, Tashka Koleva**
**Komplex Nadejda-4 Bl. 435-A**
**Sofia(BG)**
Erfinder: **Nakov, Anton Ivanov**
**Kiril i Metodi-Strasse Bl. A-B**
**Razgrad(BG)**
Erfinder: **Georgieva, Zvetanka Borissova**
**Quartal Gorublyane Vitosha-Strasse 23a**
**Sofia(BG)**
Erfinder: **Makedonska, Vanya Borissova**
**N. Mirtchev-Strasse 27-4**
**Sofia(BG)**
Erfinder: **Stantcheva, Zvetanka Nikolova**
**Komplex Druzba Bl. 97 App. 20**
**Sofia(BG)**
Erfinder: **Mondeshka, Donka Minkova**
**Rakovski-Strasse 125**
**Sofia(BG)**
Erfinder: **Hristova, Sabka Hristova**
**Beli-Lom-Strasse 3-2 App. 38**
**Razgrad(BG)**
Erfinder: **Todorova, Dimitra Zoneva**
**G. Dimitrov-Strasse 113-A**
**Razgrad(BG)**
Erfinder: **Valtcheva, Emilia Nikolova**
**Komplex 9 Sept. Bl. 3 App. 10**
**Razgrad(BG)**
Erfinder: **Ivanov, Hristo, Ivanov**
**Rakovski Str. 150**
**Sofia(BG)**
Erfinder: **Vitkova, Snejana Georgieva**
**N. Kamenov-Strasse 252/4/76**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Verfahren zur Herstellung von Syn-Aminothiazolyl- und Syn-Aminooxazolyl-Abkömmlingen von Alkoxyminoessigsäuren.**

(57) Es ist ein Verfahren zur Herstellung von Verbindungen der allgemeinen Formel:

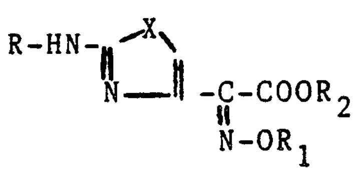

I

entwickelt, wo R für Wasserstoff oder einen niedrigen Alkyl-, Acetyl-, β,β,β-Trichlorethylcarbonyl-, Chloracetyl-oder Tritylrest steht, $R_1$ einen niedrigen Alkylrest, $R_2$-Wasserstoff oder einen niedrigen Alkylrest bedeutet und X für Schwefel oder Sauerstoff steht; als organische Phase, wo die Alkylierung erfolgt - halogenierte Kohlenwasserstoffe, Ester von niedrigen Fettsäuren, Benzol usw. eingesetzt werden. Die wässrige Phase, die mit der organischen Phase gemischt ist, stellt eine 10 - 50%ige wässrige Lösung einer

EP 0 392 037 A1

anorganischen Base dar.

Die erhaltenen Syn-Aminothiazolyl- und Syn-Aminooxazolyl-Abkömmlinge der Alkoxyiminoessigsäuren dienen als Zwischenprodukte zur Herstellung von Laktamantibiotika.

# VERFAHREN ZUR HERSTELLUNG VON SYN-AMINOTHIAZOLYL-UND SYN-AMINOOXAZOLYL-ABKÖMMLINGEN VON ALKOXYIMINOESSIGSÄUREN

Die Erfindung betrifft ein Verfahren zur Herstellung von Syn-Aminothiazolyl und Syn-Aminooxazolyl-Abkömmlingen der Alkoxyiminoessigsäuren, die als Zwischenprodukte zur Herstellung von β-Laktamantibiotika Verwendung finden.

Es sind Verfahren zur Herstellung von Aminothiazolyl- und Aminooxazolyl-Abkömmlingen der Alkoxyessigsäuren bekannt (Patent GB 1 536 282, Patent GB 2 106 519 A). Dabei wird in den meisten Fällen ein Gemisch aus Syn- und Anti-Isomeren erhalten, wobei aus diesem Gemisch die Syn-Form nur mit niedriger Ausbeute isoliert wird. Allerdings werden zur Herstellung von β-Laktamantibiotika mit hoher biologischer Aktivität nur die Syn-Isomeren gebraucht, da die Anti-Isomeren nur Produkte mit niedriger biologischer Aktivität - 25 bis 100 Hundert mal kleiner - ergeben.

Der Erfindung liegt die Aufgabe zugrunde, ein selektives Verfahren zur Herstellung von Syn-Aminothiazolyl- und Syn- Aminooxyzolyl-Abkömmlingen von Alkoxyessigsäuren mit hoher Ausbeute zu entwickeln.

Gegenstand der Erfindung ist ein Verfahren zur Herstellung von Verbindungen der allgemeinen Formel:

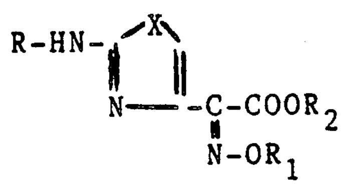

I

worin R für Wasserstoff oder ein niedriges Alkyl, Acetyl, β,β,β-Trichlorethoxycarbonyl, Chloracetyl oder den Tritylrest steht,
$R_1$ einen niedrigen Alkylrest bedeutet,
$R_2$ Wasserstoff oder einen niedrigen Alkylrest bedeutet,
$R_1$ gleich oder verschieden von $R_2$ ist und X für Schwefel oder Sauerstoff steht, das dadurch gekennzeichnet ist,
daß man Verbindungen der allgemeinen Formel:

$Y\text{-}CH_2\text{-}CO\text{-}$ C−CO $OR_2$ II
N−OH

worin $R_2$' die obige Bedeutung besitzt und Y Wasserstoff oder Halogen bedeutet, mit Dialkylsulfat in Anwesenheit von Katalysatoren der Zwischenphasen-Übertragung in einem Gemisch aus Wasser und organischem Lösungsmittel alkyliert, dann mit Thiocarbamiden oder Carbamiden kondensiert und die so erhaltenen Aminothiazolyl- und Aminooxazolyl-Abkömmlinge der 2-Methoxyiminoessigsäure der allgemeinen Formel:

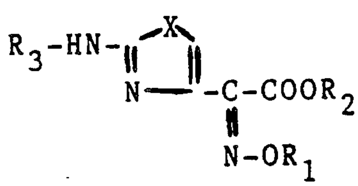

III

worin $R_1$, $R_2$ und X die obige Bedeutung besitzen und wobei $R_3$ für Wasserstoff, ein niedriger Alkyl, Acetyl, β,β,β-Trichlorethoxycarbonylrest steht, im Fall wenn $R_3$ Wasserstoff bedeutet, nach Hydrolyse silyliert, die Silylester mit Tritylchlorid oder Chlorazetylchlorid bis zum Erhalt von Verbindungen der allgemeinen Formel:

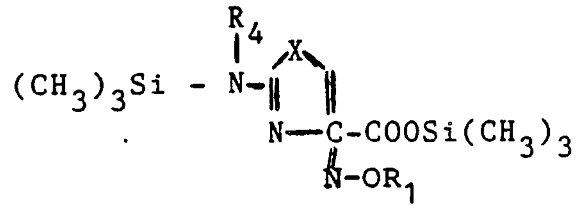

IV

reagieren läßt,
worin X die obige Bedeutung besitzt und $R_4$ einen Trityl-oder Chlorazetylrest bedeutet, welcher mit Wasser oder niedrigem aliphatischen Alkohol hydrolisiert wird.

Als phasenübertragende Katalysatoren werden z.B. quaternäre Ammonium- oder Phosphoniumsalze wie: Benzyltriethylammoniumchlorid (TEBAX), Benzyltrimethylammoniumchlorid, Tetrabutylammoniumbromid (TBAB), Methyltrioctylammoniumchlorid (Aliquat 336), Trimethyloctadecylammoniumbromid, Trialkyl ($C_8$-$C_{10}$) Methylammoniumchlorid verwendet. Als Katalysatoren können auch einige Alkylamine oder Tetraalkyldiamine eingesetzt werden.

Bei geeignetem Dosieren des Alkylierungsmittels und der Katalysatoren des Zwischenphasenübertragens verläuft die Methylierung bei energischem Rühren und einer Temperatur von -5 bis 50° C für 5 bis 60 min. Das Verfahren wird mit Hilfe der Dünnschichtchromatographie kontrolliert.

Die Menge des eingesetzten Katalysators beträgt von 0,01 bis 0,015 Mol pro 1 Mol nicht methyliertes Produkt, wobei immer der Katalysator, der am leichtesten zugänglich und billig ist, bevorzugt wird.

Die organische Phase kann ein halogenierter Kohlenwasserstoff wie Methylenchlorid, Chloroform, Tetrachlormethan, Ester der niedrigen Fettsäure wie Ethylacetat, Butylacetat, niedrige Dialkylketone wie Aceton, Ethylmethylketon, Benzol, Toluol usw. sein.

Als wässrige Phase kann man 10 bis 50 %ige Lösungen von Natriumcarbonat, Kaliumcarbonat, Natrium- oder Kaliumlauge und andere wässrige Lösungen von anorganischen Basen verwenden.

Die Kondensation mit den Thiocarbamiden, bzw. Carbamiden erfolgt in Wasser, in einem mit Wasser mischbaren organischen Lösungsmittel oder in einem inerten organischen Lösungsmittel.

Geeignete organische Lösungsmittel sind Alkohole wie Methanol, Ethanol, Isopropanol; Ketone wie Aceton, Ethylmethylketon; cyclische Ether wie Dioxan, Tetrahydrofuran; Acetonitril; halogenierte Kohlenwasserstoffe wie Chloroform, Methylenchlorid, Tetrachlormethan; Ester von niedrigen Fettsäuren wie Dimethylformamid und Dimethylacetamid.

Die Kondensation erfolgt bei Temperaturen von -10° C bis zur Siedetemperatur des Reaktionsgemisches.

Die Herstellung von Silylestern erfolgt in einem aprotischen organischen Lösungsmittel wie halogenierten Kohlenwasserstoffen, Acetonitril, Tetrahydrofuran, Dioxan, Dimethylformamid, Dimethylacetamid usw. Für die Silylierung verwendet man zweckmäßig eines der am häufigsten gebrauchten Silylierungsmittel wie Dimethyldichlorsilan, Trimethylchlorsilan, N,N-bis-Trimethylsilylcarbamid, N,o-bis-Trimethylsilylacetamid, N-Trimethylsilylcaprolaktam oder Hexamethyldisilazan. Als chlorwasserstoffentfernendes Mittel werden zweckmäßig organische Basen wie Triethylamin, Dimethylanilin, N-Methylpyrrolidin oder Gemische aus N-Silylabkömmlingen und die entsprechenden Amide oder Carbamide und Triethylammoniumchlorid verwendet.

Die Umsetzung der Silylester mit Tritylchlorid oder Chloracetylchlorid erfolgt bei Temperaturen von -10 bis 20° C , wobei eine Temperatur bei 0° C bevorzugt wird. Die Zeit für die Acylierung variiert von 90 min. bis 3 h. Tritylchlorid oder Chloracetylchlorid werden vorzugsweise im Überschuß von 0,1 bis 0,2 Mol zudosiert.

Das Verfahren wird mit Hilfe der Dünnschichtchromatographie verfolgt.

Die Hydrolyse der so erhaltenen Verbindungen der Formel IV erfolgt in quantitativer Ausbeute bei den für den Aminothiazolylring günstigsten Bedingungen unter der Einwirkung einer äquivalenten Menge Wasser oder niedrigem aliphatischen Alkohol bei 0 bis 20° C.

Die Vorteile des erfindungsgemäßen Verfahrens sind, daß man selektiv Syn-Aminothiazolyl- und Syn-Aminooxazolyl- Abkömmlinge der Alkoxyiminoessigsäuren mit hoher Ausbeute erhält.

Die Erfindung wird anhand folgender Beispiele erläutert.

Beispiel 1

Stufe A

In 200 ml Wasser werden 29 g (0,25 Mol) Methylacetoacetat und 18,7 g (0,27 Mol) Natriumnitrit gelöst. Die Lösung wird bis 0° C gekühlt und unter Umrühren werden tropfenweise 100 ml einer 4N-Schwefelsäure während 1 h zugefügt. Das Umrühren erfolgt noch weitere 90 min bei 0 bis 5° C. Dann wird das Reaktionsgemisch mit Methylenchlorid extrahiert. Die kombinierten organischen Extrakte werden nocheinmal mit 15 %iger wässriger Natriumcarbonatlösung extrahiert.

Zu den so erhaltenen wässrigen Extrakten werden Methylenchlorid, 2,9 g Benzyltriammoniumchlorid und 38 ml (0,4 Mol) Dimethylsulfat zugefügt. Das Reaktionsgemisch wird 30 min bei Zimmertemperatur gerührt. Danach wird die wässrige Schicht entfernt, während die organische Schicht mit Wasser gewaschen, dann mit Natriumsulfat getrocknet und unter Vakuum bis zur Entfernung des Lösungsmittels verdampft wird. Die so erhaltene ölige Masse wird im Kühlschrank aufbewahrt.

Man erhält 33,9 g Methylester der 2-Methoxyimino-3-oxobuttersäure - Synisomer. Nach Umkristallisierung aus Hexan werden Kristalle mit einem Schmelzpunkt von 68 - 69° C erhalten. Die Ausbeute beträgt 85,6 %.

| | | | |
|---|---|---|---|
| $C_6H_9NO_4$ ber.,% | C 45,28 | H 5,70 | N 8,80 |
| (159,14) gef.,% | 45,31 | 5,72 | 8,78 |

$^1H$ - KMR-Spektrum ($COCl_3$) , 100 MHz
δ(ppm) : 2,23 (3H,s) - $CH_3CO$
3,64 (3H,s) -CO-$OCH_3$
3,88 (3H,s) -N-$OCH_3$

Stufe B

Methylester der 2-Methoxyimino-3-oxobuttersäure - 15,9 g (0,1 Mol) wird unter Umrühren in 20 ml Chloroform gelöst. Die so erhaltene Lösung wird bis 45° C erwärmt und dann unter Umrühren am Rückflusskühler erhitzt. Zu dieser Lösung wird tropfenweise eine im voraus bereitete Lösung von 15,9 g Brom in 20 ml Chloroform zugefügt. Nach Hinzugabe der ganzen Menge an Brom wird das Reaktionsgemisch noch zusätzlich weitere 90 min gerührt, dann bis auf Zimmertemperatur abgekühlt und aufeinanderfolgend mit Wasser, einer gesättigten Natriumcarbonatlösung und wieder mit Wasser gewaschen. Die Chloroformlösung wird mit Magnesiumsulfat getrocknet und dann unter Vakuum destilliert bis zum vollständigen Entfernen des Lösungsmittels. Man erhält 14,5 ml eines dunkel gefärbten Öls des Rohproduktes Methylester der 4-Brom-2-metoxyimino-3-oxobuttersäure.

$^1H$ - KMR-Spektrum ($CDCl_3$) , 100 MHz
δ(ppm) : 3,70 (3H,s) , CO-$OCH_3$
4,03 (3H,s) , N-$OCH_3$
4,32 (2H,s) , -$CH_2Br$

Stufe C

Das so erhaltene ölförmige Produkt nach der in Beispiel 1 Stufe B beschriebenen Verfahrensweise aus Methylester der 4-Brom-2-methoxyimino-3-oxobuttersäure wird in 20 ml Ethylalkohol gelöst und dann langsam zu einer im voraus bereiteten und gekühlten Lösung von 7,7g (0,1 Mol) Thiocarbamid im Gemisch aus 20 ml Ethylalkohol und 40 ml Wasser eingegossen. Nach Zugabe der ganzen Menge des Öls wird das Reaktionsgemisch weitere 2 h bei Zimmertemperatur gerührt. Man kühlt und korrigiert den pH-Wert des Gemisches mit einer Lösung von Kaliumcarbonat auf einen pH-Wert 5,5 - 6 bis zum Erreichen eines beständigen pH's.

Das Reaktionsgemisch wird 2 h gerührt, kontrolliert und wiederum der pH-Wert korrigiert. Der Niederschlag wird filtriert und mit einer Mischung aus Wasser und Ethanol (1:1) gewaschen.

Man erhält 15,3 g Methylester der 2-/2-Aminothiazol-4-yl/2-methoxyiminoessigsäure-Synisomer mit einem Schmelzpunkt 167 - 169° C.

| | | | |
|---|---|---|---|
| $C_7H_9N_2O_3S$ ber.,% | C 39,06 | H 4,22 | N 14,9 |
| (215,228) gef.,% | 39,1 | 4,28 | 14,83 |

$^1H$ - KMR-Spektrum ($CDCl_3$) , 100 MHz
δ(ppm) : 3,72 (3H,s) -CO-$OCH_3$
3,83 (3H,s) -N-$OCH_3$
5,20 (2H, sehr breit) im Austausch mit $D_2O$-$NH_2$
6,43 (1H,s) 5-H vom Thiazolylring

Beispiel 2

Stufe A

Methylester der 2-methoxyimino-3-oxobuttersäure in einer Menge von 33,2 g wird hergestellt nach der Verfahrensweise wie in Beispiel 1, Stufe A beschrieben. Als Katalysator des Zwischenphasenprozesses wurde Tetrabutylammoniumbromid - 2 g verbraucht. Das Methylieren dauert 20 min. Die physikalisch-chemischen Charakteristika des so erhaltenen Methylesters der 2-methoxyimino-3-oxobuttersäure sind identisch mit denen des Produkts gemäß Beispiel 1, Stufe A.

Stufe B-C

31,8g (0,2 Mol) von 2-methoxyimino-3-oxobuttersäure werden unter Rühren in 60 ml Methylenchlorid gelöst. Die so erhaltene Lösung wird am Rückflußkühler erhitzt. Zu ihr wird während 3 h tropfenweise eine im voraus bereitete Lösung von 33 g Brom in 60 ml Methylenchlorid gegossen. Nach Zugabe der ganzen Menge Brom wird das Reaktionsgemisch zusätzlich noch zwei Stunden gerührt. Dann gießt man es in kaltes Wasser. Nach weiterem Rühren wird die wässrige Schicht entfernt, während die Methylenchloridschicht mit einer gesättigten Lösung von Natriumbicarbonat und Wasser gewaschen und dann mit Natriumsulfat getrocknet wird. Zu der Methylenchloridlösung werden unter Erhitzung am Rückflußkühler 15,4 g Thiocarbamid zugefügt. Das Gemisch wird 3 h lang erwärmt und dann in kaltes Wasser gegossen. Der pH-Wert des Reaktionsgemisches wird langsam auf 5,5 - 6 geführt. Nach Einstellen eines ständigen pH-Werts wird das Rühren noch 2 h lang fortgesetzt. Der Niederschlag wird filtriert und aufeinanderfolgend mit Wasser-Ethanol (1:1) und Hexan gewaschen. Man erhält 31 g Methylester der 2-/-2-Aminothiazol-4-yl/-2-methoxyimi noessigsäure - ein Produkt, das identisch ist nach seiner physikalisch-chemischen Charakteristika mit demjenigen, erhalten gemäß der in Beispiel 1 beschriebenen Verfahrensweise.

Beispiel 3

Stufen A-B

Zur Lösung von 7,6 g (0,1 Mol) Natriumnitrit in 100 ml Wasser werden langsam tropfenweise 16,4g (0,1 Mol) 4-Chlorethylacetoacetat gegossen. Die Lösung wird bis auf 0 - 5° C abgekühlt und unter Rühren während 30 min 40 ml Schwefelsäure zugefügt. Das Rühren dauert noch 90 min bei derselben Temperatur. Danach wird das Reaktionsgemisch mit Methylenchlorid extrahiert. Die kombinierten organischen Extrakte werden mit einer 15 %igen wässrigen Lösung von Natriumbicarbonat reextrahiert.

Zu den so erhaltenen wässrigen Extrakten werden gleiche Volumina von Methylenchlorid, 1,15 g Benzyltriethylammoniumchlorid und 15,2g (0,16 Mol) Dimethylsulfat zugesetzt. Das Reaktionsgemisch wird 30 min lang bei Zimmertemperatur gerührt, die wässrige Schicht wird abgeschieden. Man wäscht mit Methylenchlorid und entfernt diese Schicht. Die Methylenchloridlösung wird mit Wasser gewaschen, mit Natriumsulfat getrocknet und unter Vakuum bis zum Entfernen des Lösungsmittels verdampft. Man erhält 19,8 g eines dunkelgelben ölförmigen Restes, nämlich Ethylester der 4-chlor-2-methoxyimino-3-oxobuttersäure.

$^1H$ - KMR-Spektrum ($CDCl_3$), 100 MHz
δ(ppm) : 1,22 (3H,t) und 4,28 (2H,q) $-OC_2H_5$
3,88 (3H,s) $-N-OCH_3$
4,60 (2H,s) $-ClCH_2$

Stufe C

Zur Lösung von 8,4 g (0,1 Mol) Natriumacetat und 7,6 g Thiocarbamid im Gemisch Wasser-Methanol (80:80) werden tropfenweise unter Rühren 20 g des ölförmigen Produkts, hergestellt nach der Verfahrensweise wie im Beispiel 3, Stufe A-B beschrieben, zugegeben. Das Reaktionsgemisch wird am Rückflußkühler bei 45-50° C während 1 h erhitzt, danach abgekühlt und der pH-Wert bis ungefähr 6 korrigiert mit Hilfe einer gesättigten Natriumbicarbonatlösung. Nach Einstellen eines ständigen pH-Wertes wird das Reaktionsgemisch zusätzlich noch 90 min lang gerührt. Der Niederschlag wird filtriert und mit Wasser und Ethylacetat gewaschen.

Man erhält 13,9 g Ethylester der 2-/2-Aminothiazol-4-yl/-2-methoxyiminoessigsäure-Synisomer mit einem Schmelzpunkt von 161 bis 163° C. Die Ausbeute beträgt 65 %.

| | | | |
|---|---|---|---|
| $C_8H_{11}N_3O_3S$ ber.,% | C 41,91 | H 4,84 | N 18,33 |
| (229,17) gef.,% | 41,89 | 4,83 | 18,35 |

$^1H$ - KMR-Spektrum ($CDCl_3$), 100 MHz
δ(ppm) : 3,69 (3H,s) $-CO-OC_2H_5$
3,82 (2H, sehr breit) im Austausch mit $D_2O$- $NH_2$
6,40 (1H,s) - 5H-Thiazolring

Beispiel 4

Stufe A

Zur Lösung von 12,9 g (0,1 Mol) Ethylacetoacetat und 9,6 g Natriumnitrit (0,14 Mol) in 100 ml Wasser, gekühlt bis auf 0 - 5° C werden tropfenweise unter Rühren 40 ml Schwefelsäure (4N) zugegeben. Die Zugabe der Schwefelsäure erfolgt langsam während 1 h unter Rühren. Das Rühren wird noch 90 min lang unter Kühlen fortgesetzt und dann das Reaktionsgemisch mit Ethylazetat extrahiert. Die kombinierten organischen Extrakte werden mit einer gesättigten Natriumchloridlösung gewaschen und mit einer 25 %igen wässrigen Kaliumcarbonatlösung reextrahiert.

Zu den so erhaltenen wässrigen Extrakten werden Benzol, 0,8 g Tetrabutylammoniumbromid und 15,1 ml (0,16 Mol) Dimethylsulfat zugegeben. Das Reaktionsgemisch wird energisch während 20 min gerührt und danach die wässrige Schicht abgeschieden, mehrmals mit Benzol gewaschen und entfernt.

Die kombinierten organischen Schichten werden mit Wasser gewaschen, mit Natriumsulfat getrocknet und unter Vakuum bis zum Entfernen des Benzols verdampft. Der so erhaltene ölförmige Rest wird im Kühlschrank aufbewahrt. Das kristallisierte Produkt wird filtriert und in Chloroform gelöst. Zur Chloroformlösung wird Hexan zugefügt. Man erhält 14,1 g weiße Kristalle mit einem Schmelzpunkt von 69° C - Ethylester der 2-methoxyimino-3-oxobuttersäure-Synisomer.

| | | | |
|---|---|---|---|
| $C_7H_{11}NO_4$ ber.,% | C 48,55 | H 6,40 | N 8,09 |
| gef.,% | 48,57 | 6,41 | 8,00 |

$^1H$-KMR-Spektrum ($CDCl_3$), 100 MHz
δ(ppm) : 2,20 (3H,s) $-CH_3CO$
3,62 (3H,s) $-OC_2H_5$
3,73 (3H,s) $-N-OCH_3$

Stufe B

Der Ethylester der 2-methoxyimino-3-oxobuttersäure in einer Menge von 14,1 g wird unter Rühren in 20 ml Chloroform gelöst. Das Gemisch wird bis auf 5 - 10° C gekühlt und zu ihm langsam eine Lösung von 15 g Brom in 20 ml Chloroform zugefügt. Nach Zugabe der ganzen Menge Brom wird das Reaktionsgemisch zusätzlich 1h lang bei 10° C gerührt.

Nach beendeter Bromierung wird die Chloroformlösung aufeinanderfolgend mit Wasser, gesättigter Natriumcarbonatlösung und wieder mit Wasser gewaschen. Die Chloroformlösung wird mit Natriumsulfat getrocknet. Nach Entfernung des Chloroforms erhält man 19,6 g gefärbten ölförmigen Rohethylester der 4-Brom-2-methoxyimino-3-oxobuttersäure.

$^1H$ - KMR-Spektrum ($CDCl_3$), 100 MHz

δ(ppm) : 3,67 (3H,s) $-CO-OCH_3$

4,00 (3H,s) $-N-OCH_3$

4,27 (2H,s) $-CH_2Br$

Stufe C

Der so erhaltene Rohester der Ethyl-4-brom-2-methoxyimino-3-oxobuttersäure aus Stufe B wird in 100 ml Tetrahydrofuran gelöst. Die gebildete Lösung wird auf 0 - 5° C gekühlt und zu ihr unter Rühren eine Lösung von 36,2 g Natriumacetat und 12 g Thiocarbamid in 80 ml Wasser zugegeben. Das Reaktionsgemisch wird 2 h lang gerührt und dann eine Nacht bei Zimmertemperatur stehen gelassen. Nachdem mit Hilfe der Dünnschichtchromatographie das Ende der Kondensation festgestellt wurde, wird das Tetrahydrofuran unter Vakuum entfernt. Das Reaktionsgemisch wird mit Methylenchlorid extrahiert. Die kombinierten Methylenchloridextrakte werden nacheinander mit gesättigter Natriumbicarbonatlösung und Wasser gewaschen. Nach dem Trocknen mit Natriumsulfat wird die Lösung unter Vakuum konzentriert und nach Erreichen eines bestimmten Volumens wird Diisopropylether zuge geben. Man erhält 10,1 g Ethylester der 2-/2-Aminothiazol-4-yl/-2-methoxyiminoessigsäure. Nach seinen physikalisch-chemischen Charakteristiken ist das Produkt identisch mit demjenigen, gewonnen nach der entsprechenden Verfahrensweise von Beispiel 3.

Beispiel 5

Stufe A-B

Zur gekühlten Lösung von 16,5 g (0,1 Mol) 4-Chlorethylacetoacetat in 60 ml Essigsäure wird langsam tropfenweise während 45 min eine Lösung von 7,6g (0,11 Mol) Natriumnitrit in 20 ml Wasser zugefügt. Das Gemisch wird zusätzlich noch 60 min gerührt und dann in eiskaltes Wasser gegossen und mit Ethylacetat extrahiert. Das Ethylacetatextrakt wird mit gesättigter Natriumchloridlösung gewaschen und dann mit 30 %iger Natriumlaugelösung reextrahiert.

Zum so erhaltenen wässrigen Extrakt werden Chloroform, 1,1 g Tetrabutylphosphoniumchlorid und 15,2 ml (0,16 Mol) Dimethylsulfat zugegeben. Das Reaktionsgemisch wird energisch gerührt während 20 min bei 0° C und danach die wässrige Schicht entfernt. Die Chloroformlösung wird mit Wasser gewaschen, mit Natriumsulfat getrocknet und unter Vakuum verdampft, um das Lösungsmittel zu entfernen. Man gewinnt 18,8 g eines ölförmigen Rests - Ethylester der 4-chlor-2-methoxyimino-3-oxobuttersäure-Synisomer.

Stufe C

Die Lösung von 17,6 g (0,085 Mol) Ethylester der 4-Chlor-2-methoxyimino-3-oxobuttersäure in 250 ml Methylethylketon, 20,4 g (0,34 Mol) Carbamid und 2,7 g (0,34 Mol) Zinkoxid wird am Rückflußkühler während 24 h erhitzt und danach das Gemisch bis auf Zimmertemperatur abgekühlt. Es folgt eine Filtrierung zur Beseitigung des nicht reagierten Carbamids und das Lösungsmittel wird unter Vakuum entfernt, bis man ein dunkles Öl erhält, welches in Methylenchlorid gelöst wird. Die Methylenchloridlösung wird mit Wasser gewaschen, mit Natriumsulfat getrocknet und unter Vakuum bis zum minimalen Volumen konzentriert. Nach Zugabe von Diisopropylether fällt ein Niederschlag von Ethylester der 2-/-2-

Aminooxazol-4-yl/-2-methoxyiminoessigsäure aus. Der Niederschlag wird filtriert und mit Diisopropylether gewaschen. Man erhält 6,5 g eines Produkts mit einem Schmelzpunkt von 139 - 141° C.

| | | | |
|---|---|---|---|
| $C_8H_{11}N_3O_4$ ber.,% | C 45,07 | H 5,20 | N 19,71 |
| (213,19) gef.,% | 45,10 | 5,27 | 19,52 |

IR (KBr) : 3438, 3181, 1701, 1680, 1588 $cm^{-1}$

Beispiel 6

Zu einer Suspension von 2,2 g (11 Mol) 2-/2-Aminothiazol-4-yl/-2-methoxyiminoessigsäure in 15 ml Methylenchlorid werden 2,5 ml (12 nMol) Hexamethyldisiloxan zugegeben. Das Gemisch wird am Rückflußkühler 4 h erhitzt und dann bis auf -5° C abgekühlt. Zum abgekühlten Reaktionsgemisch werden 1,0 ml (1,3mMol) Chloracetylchlorid zugefügt. Das Reaktionsgemisch wird 30 min bei 0-5° C gerührt und danach läßt man die Temperatur bis auf Zimmertemperatur ansteigen und rührt zusätzlich noch 2 h.

Die Azylierung wird mit Hilfe der Dünnschicht-Chromatographie verfolgt. Nach Beenden des Prozesses werden zum Reaktionsgemisch 20 ml Wasser zugefügt und der pH-Wert wird bis auf 8-8,5 geführt. Die wässrige Schicht wird abgeschieden. Dazu werden 6 ml Isopropylalkohol gegeben. Das Gemisch wird jetzt bis auf 0-5° C abgekühlt und mit Salzsäure auf einen pH-Wert von 1,5 - 2 eingestellt. Der dann erhaltene Bodensatz wird filtriert und mit Wasser und Isopropylalkohol gewaschen. Man erhält 2,94 g Syn 2-/2-Chloracetylaminothiazol-4-yl/-2-methoxyiminoessigsäure.

| | | | |
|---|---|---|---|
| $C_8H_8N_3ClO_4$ ber.,% | C 34,60 | H 2,90 | N 15,13 |
| (276,67) gef.,% | 34,57 | 2,87 | 15,17 |

$^1H$ - KMR-Spektrum ($CDCl_3$) , 100 MHz
δ(ppm) : 3,91 (3H,s) $-NOCH_3$
4,22 (2H,s) $-COCH_2Cl$
7,33 (1H,s) -5H des Thiazolrings
11,50 (2H,s sehr breit) wird mit $D_2O$-NH + COOH getauscht

Beispiel 7

Zu einer Suspension von 2,2 g (11mMol) 2-/2-Aminothiazol-4-yl/-2-methoxyiminoessigsäure in 15 ml Chloroform werden 33,2 ml (24mMol) Triethylamin und 3,1 ml (24 mMol) Trimethylchlorsilan zugegeben. Das Gemisch wird am Rückflußkühler erwärmt unter Rühren während 3 h und danach filtriert. Zum bis auf -5° C abgekühlten Filtrat werden 1,4 ml (10 Mol) Triethylamin und 2,8 ml (10 mMol) Tritylchlorid zugegeben. Das Reaktionsgemisch wird während 30 min gerührt bei derselben Temperatur und danach läßt man die Temperatur bis auf Zimmertemperatur ansteigen. Das Rühren dauert noch 3 h lang. Man gibt dann 15 ml Wasser zu und rührt noch 20 min. Der pH-Wert des Gemisches wird auf 1,5 einge stellt und die Schichten werden getrennt. Die wässrige Schicht wird mit einer Mischung von Chloroform und Methanol gewaschen. Die kombinierten organischen Extrakte werden mit Wasser gewaschen, 300 g Aktivkohle zugegeben, während 15 min gerührt, filtriert, getrocknet und das Lösungsmittel unter Vakuum konzentriert. So erhält man 4,7g Syn-2-/2-Tritylaminothiazolyl-4-yl/-2-methoxyiminoessigsäure.

| | | | |
|---|---|---|---|
| $C_{25}H_{21}N_3O_3S$ ber.,% | C 67,70 | H 4,77 | N 9,47 |
| (443,50) gef.,% | 67,20 | 4,78 | 9,50 |

$^1H$ - KMR-Spektrum ($COCl_3$ + DMSO-$d_6$), 100 MHz
δ(ppm) : 3,86 (3H,s) $-NOCH_3$

6,51 (1H,s) -5H des Thiazolrings
7,20 (15H,s) -3 x $C_6H_5$
9,35 (2H,s, erweitert),wird mit $D_2O$-NH + COOH getauscht

Beispiel 8

Es wird die Silylierung von 2,35 g (11 mMol) 2-/2-Aminothiazolyl-4-yl/-2-Ethoxyiminoessigsäure mit 2,5 ml (12 mMol) Hexamethyldisilizan durchgeführt und danach eine direkte Tritylierung in Acetonitril, wie in Beispiel 1 beschrieben. Das Verfahren wird mit Hilfe der Dünnschicht-Chromatographie verfolgt. Zum Schluß wird das Acetonitril unter Vakuum beseitigt. Zum Rest werden 25 ml Wasser zugegeben. Danach erfolgt die Isolierung wie im Beispiel 7 beschrieben. Man erhält 4,8 g Zielprodukt.

| | | | |
|---|---|---|---|
| $C_{26}H_{23}N_3O_3S$ ber.,% | C 68,25 | H 5,07 | N 9,18 |
| (457,52) gef.,% | 68,27 | 5,11 | 9,21 |

δ(ppm) : 4,30 (3H,s) -$NOC_2H_5$
6,53 (1H,s) -5H des Thiazolylrings
7,28 (15H,s) -3 x $C_6H_5$
9,40 (2H, erweitert), wird mit $D_2O$-NH + COCH getauscht

Beispiel 9

Zur Suspension von 2,2 g (11 mMol) 2-/2-Aminothiazol-4-yl/-2-methoxyiminoessigsäure in 15 ml Methylenchlorid werden 2,5 ml (12 mMol) Hexamethyldisilazan zugegeben. Das Gemisch wird am Rückflußkühler unter Rühren während 4 h erhitzt und danach bis auf -5° C abgekühlt. Zur abgekühlten Reaktionsmischung werden 1,4 ml (10 mMol) Triethylamin und 2,8 g (10 mMol) Tritylchlorid zugefügt. Man rührt 30 min lang bei -5° C bis 0° C und läßt dann die Temperatur bis auf Zimmertemperatur steigen, um danach noch 3 h zu rühren. Die Tritylierung wird mit Hilfe der Dünnschicht-Chromatographie verfolgt. Nach Beendigung des Verfahrens werden zur Reaktionsmischung 15 ml Wasser zugegeben. Das Reaktionsgemisch wird 20 min lang gerührt und danach bis auf einen pH-Wert von 1,5 angesäuert. Die Schichten werden abgetrennt. Die Methylenchloridschicht wird mit Wasser gewaschen, danach mit wasserfreiem Natriumsulfat getrocknet und auf einem Rotations-Vakuumverdampfer verdampft. Man erhält 4,6 g Syn 2-/2-Tritylaminothiazol-4-yl/-2-methoxyiminoessigsäure , deren physikalisch-chemischen Charakteristika identisch mit denjenigen des Produkts,erhalten in Beispiel 7,sind.

Beispiel 10

Zu 15 ml Methylenchlorid werden 2,2 g (11 mMol) 2-/2-Aminothiazol-4-yl/-2-methoxyiminoessigsäure und 2,7 ml Bistrimethylacetamid zugegeben. Das so erhaltene Gemisch wird 90 min lang am Rückflußkühler erhitzt. Danach wird das Verfahren gemäß der beschriebenen Verfahrensweise in Beispiel 1 fortgesetzt. Man erhält 4,6 g eines Produkts das identisch mit dem des Beispiels 7 ist.

Beispiel 11

Zur Suspension aus 4,0 g (20 mMol) 2-/2-Aminothiazol-4-yl/-2-methoxyiminoessigsäure in 50 ml Methylenchlorid werden unter Rühren 8,8 ml (64 mMol) Triethylamin zugegeben. Das Reaktionsgemisch wird zusätzlich noch 30 min bei Zimmertemperatur gerührt. Man fügt 3,6 g (60 mMol) Carbamid zu und danach wird das Gemisch bis auf 5° C gekühlt und unter Rühren werden tropfenweise 88 ml (64 mMol) Trimethylchlorsilan zugefügt. Das Reaktionsgemisch wird 30 min lang gerührt bei Zimmertemperatur und zwei Stunden unter Erhitzen am Rückflußkühler. Man kühlt bis auf -5° C. Dann werden 2,8 ml (20 mMol) Triethylamin und 5,6g (20 mMol) Tritylchlorid hinzugefügt. Weiterhin erfolgt das Azylieren und danach Isolierung gemäß der Verfahrensweise wie im Beispiel 7 beschrieben. Man erhält 8 g eines Produkts

identisch mit dem des Beispiels 7.

Beispiel 12

Der Ethylester der 2-/2-Aminothiazol-4-yl/-2-ethoxyiminoessigsäure in einer Menge von 3,0 g wird in 15 ml Wasser suspendiert. Die Suspension wird gekühlt und dahinein gießt man die Lösung von 1,9 g Kaliumlauge und 25 ml Wasser. Das Reaktionsgemisch wird 30 min lang gerührt unter Kühlung und 3 h bei Zimmertemperatur. Der pH-Wert wird bis zur neutralen Reaktion eingestellt. Dann extrahiert man mit Ethylazetat. Aus der wässrigen Schicht wird 2-//2-Aminothiazol-4-yl/-2-ethoxyiminoessigsäure abgeschieden. Die so erhaltene Säure wird silyliert in Methylenchlorid mit Trimethylchlorsilan in Anwesenheit von Triethylamin. Die Silylierung und die weitere Reaktion mit Tritylchlorid erfolgt gemäß dem Verfahren von Beispiel 2. Man erhält 4,11 g von 2-/2-Tritylaminothiazol-4-yl/-2-ethoxyiminoessigsäure die identisch ist mit dem Produkt des Beispiels 8.

## Ansprüche

1. Verfahren zur Herstellung von Syn-Aminothiazolyl- und Syn-Aminooxazolyl-Abkömmlingen von Alkoxyiminoessigsäuren der allgemeinen Formel:

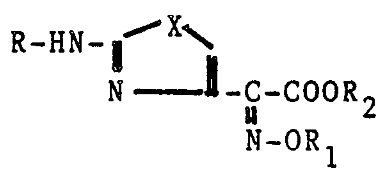

I

worin R für Wasserstoff oder niedriges Alkyl, den Acetyl, $\beta,\beta,\beta$-Trichlorethoxycarbonyl-, Chloracetyl- oder den Tritylrest steht; $R_1$ einen niedrigen Alkylrest bedeutet; $R_2$ Wasserstoff oder einen niedrigen Alkylrest bedeutet; $R_1$ gleich oder verschieden von $R_2$ ist und X Schwefel oder Sauerstoff bedeutet, **gekennzeichnet** dadurch, daß man Verbindungen der allgemeinen Formel:

$$Y\text{-}CH\text{-}CO\text{-}\underset{\underset{N-OH}{\|}}{C}-CO\ OR_2 \qquad II$$

worin $R_2$ die obige Bedeutung besitzt und Y Wasserstoff oder Halogen bedeutet, mit Dialkylsulfat in Anwesenheit von Katalysatoren der Zwischenphasenübertragung im Gemisch Wasser - organisches Lösungsmittel alkyliert und danach mit Thiocarbamiden oder Carbamiden kondensiert, wobei die so erhaltenen Aminothiazolyl- und Aminooxazolyl-Abkömmlinge der 2-Methoxyiminoessigsäure der allgemeinen Formel:

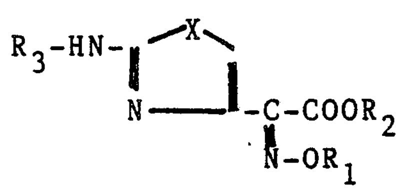

III

worin $R_1$, $R_2$ und X die obige Bedeutung besitzen und $R_3$ für Wasserstoff, einen Alkyl-, Azetyl- oder $\beta,\beta,\beta$,-Trichlorethoxycarbonylrest steht, wenn $R_3$ Wasserstoff bedeutet, nach der Hydrolyse die Silylierung erfolgt und die Silylester mit Tritylchlorid oder Chloracetylchlorid reagieren bis zum Erhalt von Verbindungen mit allgemeiner Formel:

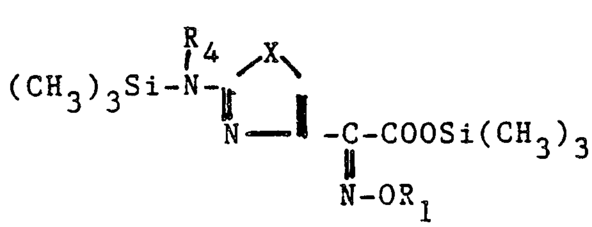

IV

worin X die obige Bedeutung besitzt und $R_4$ für den Trityl- oder Chloracetylrest steht, mit Wasser oder einem niedrigen aliphatischen Alkohol hydrolysiert.

2. Verfahren nach Anspruch 1, dadurch **gekennzeichnet,** daß als phasenübertragende Katalysatoren quaternäre Ammonium- oder Phosphoniumsalze eingesetzt werden.

3. Verfahren nach Anspruch 1, dadurch **gekennzeichnet,** daß als organische Phase im Gemisch Wasser - organisches Lösungsmittel, worin die Alkylierung erfolgt, halogenierte Kohlenwasserstoffe, Ester von niedrigen Fettsäuren, Benzol und/oder Toluol eingesetzt werden.

4. Verfahren nach Anspruch 1, dadurch **gekennzeichnet,** daß die wässrige Phase im Gemisch Wasser - organisches Lösungsmittel eine 10 - 50 %ige wässrige Lösung einer anorganischen Base darstellt.

Europäisches Patentamt

# EUROPÄISCHER RECHERCHENBERICHT

Nummer der Anmeldung

EP 89 10 6362

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 045 005 (LONZA AG)<br>* Claims *<br>--- | 1-4 | C 07 D 277/593<br>C 07 D 263/48<br>C 07 C 251/34<br>C 07 F 7/08 |
| Y | DE-C-2 759 895 (ROUSSEL-UCLAF)<br>* Insgesamt *<br>--- | 1-4 | |
| D,A | EP-A-0 076 452 (CIBA-GEIGY AG)<br>* Ansprüche 33-36 *<br>----- | 1-4 | |

RECHERCHIERTE SACHGEBIETE (Int. Cl.5)

C 07 D 277/00
C 07 D 263/00
C 07 C 251/00

Der vorliegende Recherchenbericht wurde für alle Patentansprüche erstellt

| Recherchenort | Abschlußdatum der Recherche | Prüfer |
|---|---|---|
| DEN HAAG | 08-11-1989 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument